# EUROPÄISCHE PATENTSCHRIFT

(11) **EP 3 328 482 B1**
(45) Veröffentlichungstag und Bekanntmachung des Hinweises auf die Patenterteilung: **21.08.2019**
(21) Anmeldenummer: 16747478.2
(22) Anmeldetag: 27.07.2016
(51) Int. Cl.: A61N 1/20, A61N 1/05

(54) **IMPLANTIERBARE GLEICHSTROMELEKTRODENANORDNUNG**
IMPLANTABLE DIRECT CURRENT ELECTRODE ASSEMBLY
SYSTEME D'ELECTRODE A COURANT CONTINU IMPLANTABLE

(30) Priorität: 31.07.2015 EP 15179354; 27.08.2015 EP 15182642
(43) Veröffentlichungstag der Anmeldung: 06.06.2018
(73) Patentinhaber: Berlin Heals Holding AG, 6300 Zug (CH)
(72) Erfinder: MÜLLER, Johannes, 10717 Berlin (DE)
(74) Vertreter: Liebetanz, Michael
(86) Internationale Anmeldenummer: PCT/EP2016/067923
(87) Internationale Veröffentlichungsnummer: WO 2017/021255

(56) Entgegenhaltungen:
- EP-A1- 0 559 932
- EP-A1- 1 870 131
- US-A- 5 649 971
- US-B2- 6 587 718
- US-B2- 7 319 898

## Beschreibung

### TECHNISCHES GEBIET

Die Erfindung betrifft eine implantierbare Gleichstromelektrodenanordnung mit zwei implantierbaren Elektroden und einer Steuerschaltung, wobei die zwei Elektroden mit der Steuerschaltung verbunden sind, wobei die Steuerschaltung ausgestaltet ist, um zwischen den beiden Elektroden eine Potentialdifferenz aufzubauen, sodass zwischen diesen Elektroden ein Gleichstrom fliessen kann.

### STAND DER TECHNIK

Eine solche Elektronenanordnung ist aus der WO 2006/106132 bekannt, die eine Elektrode zur Behandlung von organischem Gewebe mittels Gleichstrom bekannt zeigt, wobei Flächenelektroden vorgesehen sind, um über die Flächenelektroden ein möglichst großes Gewebeareal des Herzmuskels mit Gleichstrom definierter Größe zu durchströmen.

Aus dem Stand der Technik sind ferner implantierbare Elektroden bekannt, die als sogenannte Coil-Elektroden zur Defibrillation von arrhythmisch schlagenden Herzen genutzt werden. Diese Elektroden werden außerdem angewandt, um über ihre im Gewebe des Herzens verankerte Spitze und/oder einen Elektrodenring in der Nähe der Elektrodenspitze elektrische Stromimpulse abzugeben und/oder über diese das EKG zur Steuerung des Implantats zu detektieren (Sensingfunktion der Elektrode). Dabei kennt der Stand der Technik solche Elektroden insbesondere dahingehend, dass sie als Herzschrittmacherelektroden, als pulsgebende Elektroden oder Sensingelektroden vorgesehen sind. Als Gegenelektrode wird häufig das Gehäuse des Implantats genutzt (unipolare Stimulation, unipolares Sensing). Dabei ist es von Vorteil, wenn die Coil-Elektrode endokardial rechts innen in die Herzkammer vorgeschoben wird. Solche Coil-Elektroden sind mit einer in den Herzmuskel verankerbaren Spitze versehen, um dann den Stromimpuls über diese Spitze oder einen Elektrodenring in der Nähe der Spitze in das damit verbundene Gewebe mit dem Ziel weiterzugeben, die elektrische Erregung des Herzens zu erhöhen (Herzfrequenzerhöhung) und/oder die elektrische Erregung des Herzens verbessern (zu synchronisieren; CRT-Funktion) und/oder das EKG des Herzens zu detektieren. Es ist aus dem Stand der Technik ferner bekannt, als Gegenelektrode zu der Coil-Elektrode eine weitere Elektrode in den Koronarsinus, also Vene des Koronarsystems des Herzens zu platzieren (bipolare Stimulation). Somit ist es möglich, auch über eine solche Elektrodenanordnung das Herz mit dem Ziel elektrisch zu erregen, die Herzfrequenz zu erhöhen, die elektrische Erregungsausbreitung im Herzen zu synchronisieren und/oder ein bipolares Sensing für die Steuerung oder Regelung des Implantats zu nutzen. Der Vorteil einer bipolaren Elektroden Konfiguration liegt häufig in einem besseren Sensing- und/oder Stimulationsverhalten mit einem geringeren Energieverbrauch.

US 7,319,898 B2 offenbart ein System umfassend einen Defibrillator mit einem selbstadaptierenden Defibrillatorinduktionsmerkmal, um die Wirksamkeit des Defibrillators beim Erfassen und Beenenden der Fibrillation eines Herzens zu bewerten. Eine Anzahl von Coilelektroden sind im Herzen angeordnet, um diese Funktion zu erreichen.

US 6,587,718 B2 bezieht sich auf eine iontophoretische Abgabe eines Therapeutikums an Herzgewebe, wobei das Therapeutikum einer ersten Seite des Herzgewebes zugeführt wird. Eine Elektrode ist perkutan auf einer zweiten Seite des Herzgewebes angeordnet und wird mit Energie versorgt, um das Therapeutikum in das Herzgewebe zu migrieren.

Weitere Dokumente des technischesn Hintergrundes im Stand der Technik sind die EP 1 870 131 A1 für eine ventrikulare eine Fibrillation verhindernde Vorrichtung, EP 0 559 932 A1 für eine implantierbare Anordnung für das Defibrillieren eines Herzens unter Einsatz einer planaren Elektrode und US 5,649,971 A für eine weitere Vorrichtung und Verfahren zum Induzieren einer Fibrillation.

### DARSTELLUNG DER ERFINDUNG

Ausgehend von diesem Stand der Technik liegt der Erfindung die Aufgabe zugrunde, eine Elektrodenanordnung anzugeben, um die Gleichstromwirkung zur Heilung des erkrankten Herzmuskels zu verbessern und insbesondere die Behandlungssicherheit zu erhöhen.

Erfindungsgemäss wird die Aufgabe mit einer Elektrodenanordnung mit den Merkmalen des Anspruchs 1 erfüllt. Die eine Elektrode ist eine Coil-Elektrode mit einer durch den Abstand zwischen Trikuspidalklappe und dem Trikuspidalklappe und Pulmonalklappe gegenüberliegenden Ende der rechten Herzkammer vorbestimmten maximalen Länge, was bedeutet, dass bei der implantierbaren Gleichstromelektrodenanordnung gemäss der Erfindung Coil-Elektroden vorbestimmter Länge vorgesehen sind, wobei der Operateur vorteilhafter eine Coil-Elektrode auswählt, die den besagten Abstand möglichst ausfüllt. Aber auch kürzere Elektroden können eingesetzt wird, wobei die Länge der Coil-Herzkammerelektrode definiert ist durch die eine leitende Metallmantelfläche beziehungsweise Coilfläche, die einen Mantel aufspannt. Sie beträgt beispielsweise in der Länge 4 bis 12 Zentimeter, insbesondere ca. 6 bis 10 Zentimeter und entweder 6 bis 7 oder 8 Zentimeter bei 10° C), und ist so ausgestaltet, um nach der Durchführung durch die rechte Herzklappe möglichst die gesamte Länge der rechten Herzkammer auszufüllen. Die die Elektrode umgebende Oberfläche sollte bei Standardanwendungen z.B. zwischen 3 und 5, insbesondere 4 bis 4,5 cm² betragen, und kann aus einer Palladium-Iridium Legierung bestehen.

Es wird damit gegenüber der bekannten Flächenelektrodenanordnung für die Anwendung von Gleichstrom eine andere Elektrodenanordnung anzugeben, die es insbesondere ermöglicht, auf minimalinvasiven Wege eine Vorrichtung zu implantieren, mit der eine Gleichstrombehandlung des Herzens möglich ist, die der Heilung des Herzmuskels dienen soll, die aber keinen Einfluss auf die Erregung bzw. die Kontraktion des Herzens hat (nicht-erregende [unterschwellige] Gleichstrombehandlung des Herzens).

Dies ist insbesondere daher von Vorteil, dass eine solche Gleichstromherzbehandlung im Schnitt einen Zeitraum von einigen Monaten bis maximal einem halben Jahr dauert und diese Zeit auch der Batterielaufzeit für eine solche Gleichstrombehandlung entspricht.

Diese Batterielaufzeit ist natürlich gegenüber der Batterielaufzeit von Herzschrittmacherelektroden stark verkürzt, da im Gegensatz zu den dortigen Pulssystemen hier ein kontinuierlich fließender geringer Gleichstrom die Batterie belastet. Ziel ist eine Behandlung für eine Zeitdauer von 6 Monaten aufwärts, bis hin zu einem Jahr. Je nach Definition von Gleichstrom kann man die Vorrichtung hier als Gleichstromelektrodenanordnung für im Wesentlichen konstanten nicht-pulsierenden Gleichstrom bezeichnen, das heisst bis auf vorbestimmte Änderungen der Stromstärke wird über Zeitdauern von Minuten bis zu mehreren Stunden ein in eine Richtung konstant fliessender Strom abgegeben, wo nach die Stromrichtung umgekehrt werden kann, um das Auftreten von störenden Elektrolyseprodukten an den Elektroden zu vermeiden. Die eingesetzten Stromstärken liegen vorzugsweise zwischen 0,001 und 10 Milliampere. Die Stromdichte liegt beispielsweise zwischen 0,01 Mikroampere pro Quadratzentimeter und 0,1 Milliampere pro Quadratzentimeter. Die Stromdichte kann vorteilhafterweise in einem Intervall von 0,1 bis 20 Mikroampere pro Quadratzentimeter, vorzugsweise zwischen 1 bis 15 Mikroampere pro Quadratzentimeter, bevorzugt zwischen 5 und 10 Mikroampere pro Quadratzentimeter gehalten werden.

Gerade daher ist es von Interesse, dass der behandelnde Arzt die Elektroden auf minimal invasivem Wege setzen kann. Ein Vorteil des Einsatzes von Coil-Elektroden gegenüber Flächenelektroden liegt darin, dass der Strom dann nicht mehr den auf dem Herzmuskel bestehenden Flüssigkeitsfilm als Weg mit dem geringeren elektrischen Widerstand (Kurzschlussstrom) für den Stromfluss zwischen den beiden Elektroden benutzen kann. Bei zwei auf den Herzmuskel aufgelegten Elektroden besteht diese Möglichkeit, dass der Strom über eine solche auf der Oberfläche bestehende leitfähige Schicht mit geringem elektrischem Widerstand fliesst und nicht den direkten kurzen Weg durch die Herzmuskulatur mit höherem elektrischem Widerstand zwischen den beiden Flächenelektroden benutzt.

Eine implantierbare Gleichstromelektrodenanordnung weist zwei implantierbare Elektroden und eine Steuerschaltung auf, wobei die erste und die zweite Elektrode mit der Steuerschaltung elektrisch über vorzugsweise einadrige, isolierte Leitungen verbunden sind, wobei die Steuerschaltung ausgestaltet ist, um zwischen den beiden Elektroden eine Potentialdifferenz aufzubauen, sodass zwischen den beiden Elektroden ein Gleichstrom fliessen kann, während die eine Elektrode eine Coil-Elektrode mit einer durch den Abstand zwischen Trikuspidalklappe und dem Trikuspidalklappe und Pulmonalklappe gegenüberliegenden Ende der rechten Herzkammer (Spitze des rechten Ventrikels) vorbestimmten maximalen Länge ist.

Die andere Elektrode kann alternativ eine Coil-Elektrode für den Koronarsinus sein oder eine Flächenelektrode zum Aufbringen auf der Aussenseite der linken Herzkammer. Eine Flächenelektrode hat eine leitende Oberfläche von nicht mehr als 40 cm², und die Gesamtfläche der Elektrode sollte 90 mal 60 mm nicht überschreiten. Die Flächenelektrode kann insbesondere eine im wesentlichen rechteckige bis leicht trapezförmige Fläche sein, also eine Verringerung der Breite von beispielsweise 8 cm auf unter 6 cm über eine Länge von 6,5 bis 8 cm, wobei auf der grösseren Seite des Trapezes nach ca. 1/3 der Länge ein beidseitiger V-förmiger Einschnitt bis auf unter 3 bis 4 cm vorgesehen ist, so dass sich neben dem zentralen Bereich auf jeder Seite zwei flexible Lappen ausbilden. An der schmaleren Trapezseite ist eine Lasche für eine aufsetzbare elektrische Zuleitung vorgesehen, die an diese Öffnung herangebracht und eingeklipst wird.

Vorteilhafterweise besteht die erste Coil-Elektrode ausschliesslich aus einer oder mehreren Wendeln, so dass dort kein komplizierter Aufbau mit Sensoren oder einer implantierbaren Spitze besteht. Dabei ist distale Ende der ersten Coil-Elektrode vorzugsweise stumpf, um sicherzustellen, dass diese Elektrode nur in der Spitze des rechten Ventrikels zu liegen kommt und die Ventrikelwand oder das Ventrikelseptum nicht perforiert wird. Damit können die Coil-Elektroden gemäss der Erfindung eine einfache Spirale umfassen und benötigen insbesondere keine Sensing-Elektroden zur Rückkopplung von der Reaktion des Herzens. Ferner umfassen sie vorteilhafterweise auch keine mit Widerhaken versehene verankerbare Spitze. Sie sind ausgestaltet, um lose in der Herzkammer beziehungsweise im Coronarsinus zu liegen zu kommen.

Vorteilhafterweise ist die in die rechte Herzkammer einzufügende Coil-Elektrode von einem grösseren Durchmesser als bei für die Defibrillation und/oder Pulsübertragung zur Erregung mit folgender Kontraktion vorgesehenen bekannten Coil-Elektroden, da der Strom vom an der Ventrikelwand der rechten Herzkammer liegenden Elektrodenkörper über Blut und Herzmuskelgewebe zur Gegenelektrode abgegeben und nicht über die Spitze der Elektrode in das Herzmuskelgewebe übermittelt wird. Der größere Durchmesser und damit die größere stromabgebende Fläche erlauben einen höheren Strom, ohne eine vorgegebene maximale Stromdichte, die für eine optimale Therapie sinnvoll ist, zu überschreiten. Sie haben zusätzlich den Vorteil, ein größeres Gewebeareal des Herzmuskels mit Strom zu durchfluten.

Bei einem anderen Ausführungsbeispiel umfasst die Elektrodenanordnung neben der für die rechte Herzkammer vorgesehene Coil-Elektrode als zweite Elektrode eine Flächenelektrode, die auf dem linken Teil des Herzens angebracht wird. Hier besteht der Vorteil gegenüber dem Stand der Technik, dass der Weg des elektrischen Stromes durch das Herzmuskelgewebe garantiert ist, da es keinen auf der Herzoberfläche abfliessenden Strom geben. Andererseits wird durch die eine grossflächige Patch-Elektrode der Vorteil erreicht, dass sich der von der Coil-Elektrode ausgehende Strom fächerförmig ausweitet und durch einen grossen Anteil des Herzmuskelgewebes hindurch fliesst. Dies ist ganz im Sinne der Behandlung mit Gleichstrom, da im Gegensatz zum Herzschrittmacher eben keine die Herztätigkeit unterstützende Impulsgeneration zur Erregung des Herzmuskels an einem bestimmten Ort notwendig ist, sondern ein über einen langen Zeitraum abzugebender geringer Strom das Ziel des Einsatzes der Elektrodenanordnung ist, der durch die Herzmuskulatur fliesst dazu führt, dass die kranke Muskulatur regeneriert wird.

Bei der implantierbaren Gleichstromelektrodenanordnung kann die elektrische Verbindung zwischen den ersten und zweiten Elektroden und der Steuerschaltung vorteilhafterweise als isolierte, einadrige Leitungen ausgestaltet sein.

Bei einer vorteilhaften Ausgestaltung der Coil-Elektrode für die implantierbare Gleichstromelektrodenanordnung besteht diese ausschliesslich aus einer oder mehreren elektrisch leitenden Wendeln, die um einen aus einem elektrisch isolierenden Material gefertigten Kern der Elektrode gewunden sind, der einstückig mit dem isolierenden Mantel der Zuleitung verbunden ist. Der Wendeldurchmesser kann 0,10 bis 0,20 insbesondere 0,15 Millimter sein.

Vorteilhafterweise ist das distale Ende der ersten Coil-Elektrode und ggf. dass der zweiten Coilelektrode stumpf und ragt über das Ende der Wendel(n) hinaus, so dass es elektrisch isolierend auf dem Herzkammerboden im Bereich der Spitze des rechten Ventrikels aufliegen kann.

In einer anderen Ausgestaltung kann das distale Ende der ersten Coil-Elektrode aus mehreren voneinander abragenden freien Enden bestehen, die man als Widerhaken bezeichnen kann, und die über das Ende der Wendel(n) hinausragen, so dass diese Verankerungsspitzen in das Muskelgewebe und im Trabekelwerk vorgeschoben werden können, um eine elektrisch isolierte Verankerung zu gewährleisten. Dabei sind die freien Enden spitz zulaufend und/oder können hakenförmig ausgestaltet sein. Dann tritt der Strom im Wesentlichen aus der Seitenfläche der Wendeln aus, die der zweiten Elektrode gegenüberliegen und fliesst durch den mit Blutgefüllten rechten Ventrikel durch das interventrikuläre Septum zur Gegenelektrode des linken Ventrikels.

Wenn hier vom "Ende der rechten Herzkammer" die Rede ist so bezieht sich dies mit anderen Worten auf die Spitze des rechten Ventrikels.

Auf der Spitze der Coil-Elektrode kann ein separater elektrisch isolierender Anker vorgesehen sein, auf dem Verankerungsspitzen angeordnet sein können. Vorteilhafterweise ist der Anker hohlzyldindrisch und ist passiv mit der in ihn reichenden Elektrode verbunden, beispielsweise verklebt. Dabei kann die Elektrode in dem Anker bis zu einer in diesen eingesetzte Endkappe geführt sein, die selber hohlzylindrisch sein kann, um die Elektrodendrahtspitze aufzunehmen. Ein Zweck der Endkappe ist es dann, das distale Ende des Leiters elektrisch isolierend und mechanisch abzudecken, letzteres, um ein Eingreifen eines eingeführten Mandrins durch den Anker von innen durch ihn hindurch nach aussen zu vermeiden.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: eine skizzenhafte Darstellung eines Herzens mit einer Elektrodenanordnung gemäss einem ersten Ausführungsbeispiel der Erfindung,
- Fig. 2: eine skizzenhafte Darstellung eines Herzens mit einer Elektrodenanordnung gemäss einem zweiten Ausführungsbeispiel der Erfindung,
- Fig. 3: eine skizzenhafte Darstellung einer Coil-Elektrode gemäss einem weiteren Ausführungsbeispiel der Erfindung zum Einsatz als Herzkammerelektrode nach Fig. 1 oder Fig. 2,
- Fig. 4: eine skizzenhafte Darstellung einer anderen Coil-Elektrode gemäss einem weiteren Ausführungsbeispiel der Erfindung zum Einsatz als Herzkammerelektrode nach Fig. 1 oder Fig. 2,
- Fig. 5: eine skizzenhafte Darstellung einer Ankerspitze einer Coil-Elektrode gemäss einem Ausführungsbeispiel der Erfindung zum Einsatz als Herzkammerelektrode nach Fig. 1 oder Fig. 2, und
- Fig. 6: einen Querschnitt durch die Ankerspitze entlang der Linie VI-VI in Fig. 5, welche auf eine Coil-Elektrodenspitze aufgesetzt ist.

### BESCHREIBUNG BEVORZUGTER AUSFÜHRUNGSFORMEN

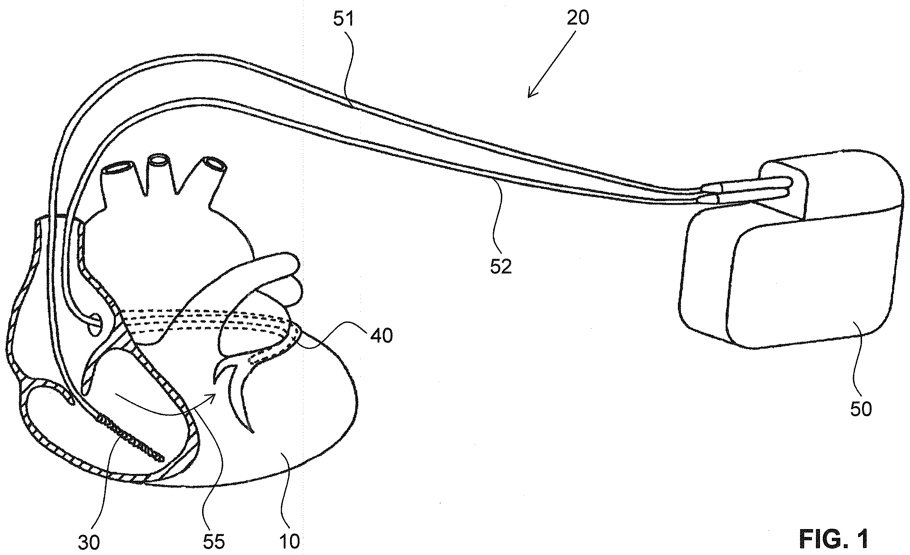

Fig. 1 zeigt eine skizzenhafte Darstellung eines Herzens 10 mit einer Elektrodenanordnung 20 gemäss einem ersten Ausführungsbeispiel der Erfindung. Die implantierbare Gleichstromelektrodenanordnung 20 umfasst zwei implantierbare Elektroden 30 und 40 sowie eine Steuerschaltung 50, üblicherweise in einem getrennten Gehäuse angeordnet, in dem ebenfalls die Batterie für die Stromversorgung vorgesehen ist.

Die zwei Elektroden 30 und 40 sind über zwei einadrige Kabel 51 und 52 mit der Steuerschaltung 50 verbunden.

Die Steuerschaltung 50 ist ausgestaltet, um zwischen den beiden Elektroden 30 und 40 eine Potentialdifferenz aufzubauen, sodass zwischen diesen Elektroden 30 und 40 ein Gleichstrom fliessen kann.

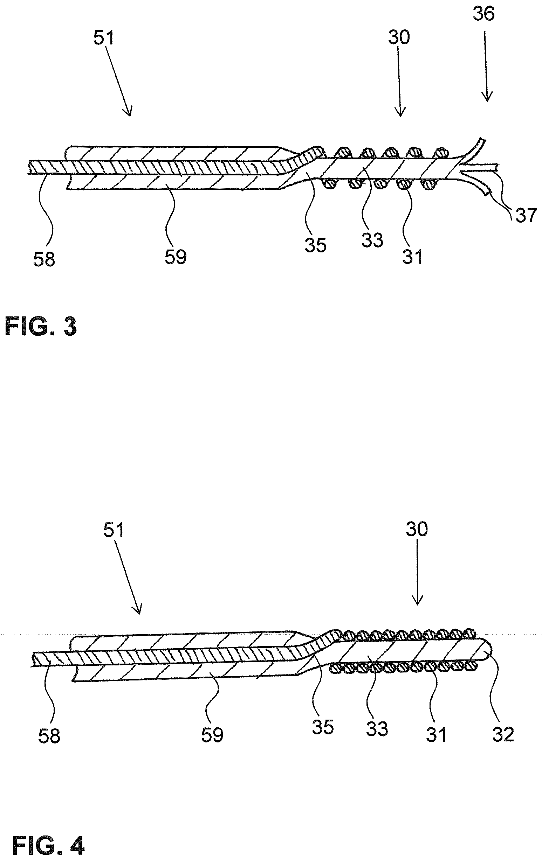

Die eine Elektrode 30 ist eine Herzkammerelektrode, vorgesehen zur Positionierung in der rechten Herzkammer und ist als Coil-Elektrode ausgestaltet. Insofern wird sie in der Folge als Coil-Herzkammerelektrode 30 bezeichnet. Die Länge der Coil-Herzkammerelektrode 30, definiert durch den eine leitende Metallmantelfläche beziehungsweise Coilfläche, die einen Mantel aufspannt, beträgt ca. 8 bis 10 Zentimeter, und ist so ausgestaltet, um nach der Durchführung durch die rechte Herzklappe möglichst die gesamte Länge der rechten Herzkammer auszufüllen: Dabei wird die Coil-Herzkammerelektrode 30 lose in die rechte Herzkammer eingelegt, sie kann aber die Wand des rechten Ventrikels berühren. Hierbei kann von Vorteil eine hier später beschriebene Elektrode nach Fig. 4 verwendet werden.

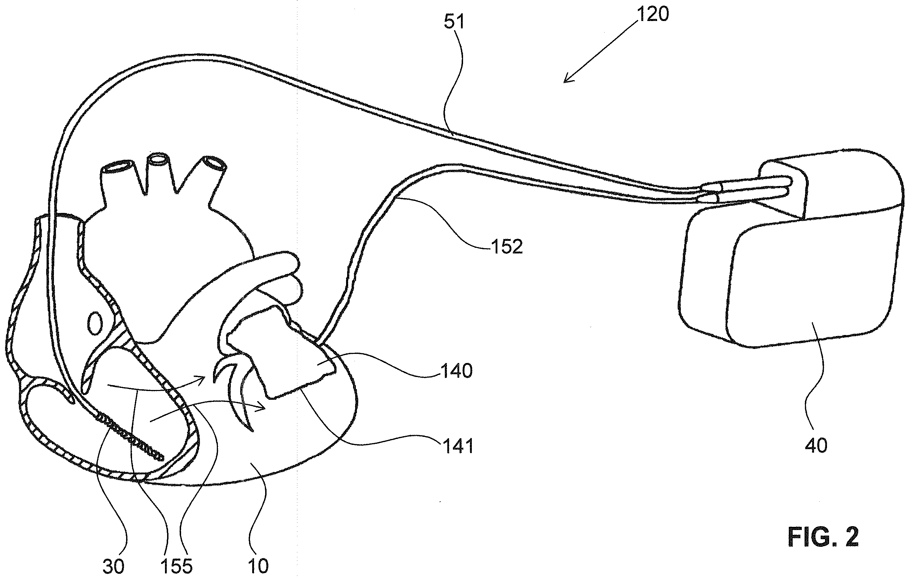

Aus den Figs. 1 und 2 scheint die Elektrode 30 in der rechten Herzkammer frei zu flottieren. Dies ist nur scheinbar so, da es sich um eine skizzenhafte 2D-Darstellung handelt. In der Regel wird sich die Elektrode 30 an die Ventrikelwand anschmiegen, in der Darstellung der Fig. 2 könnte das die dort nicht sichtbare hintere Wand sein. Die Elektrode 30 ist biegsam, um diese leichten Krümmungen anzunehmen, die in Bezug auf die longitudinale Achse weniger als 30 Grad ausmachen.

Die andere Elektrode 40 ist eine Coronarsinuselektrode, vorgesehen zur Positionierung im Cornoarsinus und ist ebenfalls als Coil-Elektrode ausgestaltet. Diese Coronarsinus-Coilelektrode 40 weist einen kleineren Durchmesser als die Coil-Herzkammerelektrode 30 auf, da sie weit in den Coronarsinus vorgeschoben werden soll, um dann in dem sich dort verengenden Endbereich zum Liegen zu kommen. Diese liegt somit an durch die Gefässwände im wesentlichen fest vorgegebener Position, die ansonsten der Operateur durch den Vorschub in longitudinaler Richtung festlegt.

Bei einer Beaufschlagung der beiden Elektroden 30 und 40 mit einer Potentialdifferenz durch die Steuerschaltung 50, vermittelt über die gegenüber der Umgebung isolierten Anschlussdrähte und -Kabel 51 beziehungsweise 52, fliesst dann ein Gleichstrom entsprechend dem Pfeil 55 durch den Herzmuskel. Dabei kann, durch die Kontrollschaltung vorbestimmt, für eine vorbestimmte Zeit zwischen einigen Minuten bis einigen Stunden die Elektrode 30 die Kathode sein, womit die Stromrichtung vorgegeben ist. Die Steuerschaltung kann dann nach einer entsprechend vorbestimmten Zeit die Stromrichtung wechseln, womit die Elektrode 40 die Kathode wird. Dabei kann sich auch die Stromstärke ändern, da der Widerstand zwischen den beiden Elektroden 30 und 40 stromrichtungsabhängig ist. Bei einem weiteren Ausführungsbeispiel steuert die Kontrolleinrichtung die Stromstärke auf einen gleichen vorbestimmten Wert.

Zwei Ausführungsformen von Coil-Elektroden 30 zum Einsatz bei dieser Anordnung 20 sind in grösserem Detail in den Fig. 3 und 4 dargestellt. Obwohl die Elektroden in diesen Figuren immer das Bezugszeichen 30 tragen, sind sie, verkleinert auch als Elektroden 40 einsetzbar, insbesondere die Elektrode nach Fig. 4.

Die Fig. 2 zeigt eine skizzenhafte Darstellung eines Herzens 10 mit einer Elektrodenanordnung 120 gemäss einem zweiten Ausführungsbeispiel der Erfindung. Die implantierbare Gleichstromelektrodenanordnung 120 umfasst zwei implantierbare Elektroden 30 und 140 sowie eine Steuerschaltung 50.

Gleiche Merkmale sind mit gleichen Bezugszeichen, ähnliche Merkmale mit entsprechend ähnlichen Bezugszeichen versehen.

Die Steuerschaltung 50 kann gleich wie in Fig. 1 beschrieben ausgestaltet sein. Auch sind die zwei Elektroden 30 und 140 sind über zwei einadrige Kabel 51 und 52 mit der Steuerschaltung 50 verbunden.

Die Steuerschaltung 50 ist auch hier so ausgestaltet, um zwischen den beiden Elektroden 30 und 140 eine Potentialdifferenz aufzubauen, sodass zwischen diesen Elektroden 30 und 140 für eine vorbestimmte Zeit von mehreren Minuten, z.B. 5 Minuten, bis mehreren Stunden, z.B. 3 Stunden, ein Gleichstrom fliessen kann. Nach dieser Zeitdauer wird die Polarität der Spannungsversorgung umgestellt, so dass sich über die Zeit keine wesentlichen Rückstände gemäß den elektrochemischen Reaktionen an den Elektroden und im Gewebe bilden können. Der Gleichstrom kann eine Grösse zwischen

Die eine Elektrode 30 ist wiederum eine Herzkammerelektrode, vorgesehen zur Positionierung in der rechten Herzkammer. und ist als Coil-Elektrode ausgestaltet. Insofern wird sie auch hier als Coil-Herzkammerelektrode 30 bezeichnet. Die Länge der Coil-Herzkammerelektrode 30, definiert durch den eine leitende Metallmantelfläche beziehungsweise Coilfläche, die einen Mantel aufspannt, beträgt ca. 6 bis 9 Zentimeter und ist so ausgestaltet, um nach der Durchführung durch die rechte Herzklappe möglichst die gesamte Länge der rechten Herzkammer in der Längsachse auszufüllen: Dabei wird die Coil-Herzkammerelektrode 30 lose in die rechte Herzkammer eingelegt, wobei sie distal passiv verankert ist und an der Ventrikelwand oder am Septum anliegen kann.

Die andere Elektrode 140 ist eine Flächenelektrode, vorgesehen zur Positionierung auf dem Herzmuskel. Sie kann beispielsweise gemäss der Lehre der US 2008/0195163 A1 ausgestaltet sein. Diese Flächenelektrode 140 wird auf der linken Seite des Herzmuskels, epikardial gegenüber der rechten Herzkammer aufgebracht.

Bei einer Beaufschlagung der beiden Elektroden 30 und 140 mit einer Potentialdifferenz durch die Steuerschaltung 50, vermittelt über die gegenüber der Umgebung isolierten Anschlussdrähte und -Kabel 51 beziehungsweise 52, fliesst dann ein Gleichstrom entsprechend den Pfeilen 155 durch den Herzmuskel. Dieser Stromfluss ist hier durch zwei Pfeile symbolisiert, da der Stromfluss hier von einer im wesentlichen längsdimensionalen Fläche von der im wesentlichen länglich orientierten Oberfläche der Coil-Elektrode 30 sich auffächernd zu der Flächenelektrode 140 ausbreitet und damit einen Fächer überstreicht. Körperlich gesehen durchfliesst der Gleichstrom ein Prisma; also ausgehend von einer Kante (des Prismas) zu seiner Grundfläche an der Patchelektrode.

Definitionsgemäss ist ein Prisma ein geometrischer Körper, dessen Seitenkanten parallel und gleich lang sind und der ein Vieleck als Grundfläche hat. Es entsteht durch Parallelverschiebung eines ebenen Vielecks entlang einer nicht in dieser Ebene liegenden Geraden im Raum und ist daher ein spezielles Polyeder. Hier ist die Gerade durch die Längsachse der Coil-Elektrode 30 vorgegeben und das Vieleck ist ein Dreieck mit der Spitze an der Coil-Elektrode 30 und einer Grundseite, die der Breite der Flächenelektrode 140 entspricht. Sofern diese Seitenränder 141 der Flächenelektrode 140 nicht parallel zur Coil-Elektroden-Ausrichtung zu liegen kommen, ist es ein verdrehtes Prisma. In allen Fällen wird von den beiden Elektroden 30 und 140 ein nicht unmassgeblicher Raumkörper aufgespannt, der garantiert, dass der von der Steuerschaltung 50 abgegebene Gleichstrom durch einen ebenfalls nicht unmassgeblichen Teilbereich des linken und in etwas verminderten Umfang auch des rechten Herzmuskels fliesst. Die Beschreibung der Geometrie des Körper, durch den der Strom fließt, durch ein Prisma ist eine Näherungsbetrachtung, da man davon ausgehen kann, dass die Elektrode nicht frei flottiert, sondern an ihrer distalen Spitze passiv fixiert ist und dann an der Ventrikelwand anliegt. Die Begrezungslinien des Körpers sind dann sicher nicht gerade sondern gekrümmt und dann ergibt sich der aufgespannte Körper nur näherungsweise als ein Prisma. Wesentlich ist aber die schmale "Kante" auf der einen Seite, die durch die Coilelektrode gebildet wird und die "breite Bodenfläche" auf der anderen Seite, die durch die Patchelektrode gebildet wird.

Eine Coil-Elektrode 30 zum Einsatz bei dieser Anordnung 120 ist in grösserem Detail in der Fig. 3 dargestellt. Fig. 3 zeigt eine skizzenhafte Darstellung einer Coil-Elektrode zum Einsatz als Herzkammerelektrode bei einem Ausführungsbeispiel nach Fig. 1 oder Fig. 2. Diese Elektrode 30 weist weniger sie bildende Elemente als eine übliche Coil-Elektrode auf.

Die Zuleitung 51 verfügt über einen leitenden einadrigen Kern 58, der von einem isolierenden Mantel 59 umgeben ist. Der Mantel 59 endet in einem Bereich vor der Elektrode 30, dass das im Zusammenhang mit den Fig. 1 und 2 beschriebene lange gewendelte Ende 31. Dabei tritt der einadrige Kern 58 im Übergangsbereich 35 aus dem Mantel 59 aus. Der Elektrodenspitzenkern 33 weist einen kleineren Durchmesser als der Mantel 59 auf, vorteilhafterweise dergestalt, dass der Gesamtdurchmesser der Elektrode 30, aufgebaut aus der Wendel und dem Elektrodenspitzenkern 33, immer noch kleiner oder höchstens genau so gross wie der Mantel 59 ist.

Die Coil-Elektrode 30 der Fig. 3 weist dann eine Spitze 36 auf, insbesondere eine Spitze 36 mit drei freien Enden 37, die insbesondere in der Spitze ein Dreieck aufspannen. Auch wenn dies in der Fig. 3 nicht gut zu erkennen ist, befinden sich die freien Enden vorteilhafterweise in einer gleichen Ebene quer zur Längsachse des Elektrodenkörpers 30. Die freien Enden 37 sind einstückig mit dem Elektrodenspitzenkern 33 und dann mit dem Körper 59 der Elektrode verbunden und bestehen aus einem nicht leitenden Material. Damit kann die Elektrodenspitze 36 im Herzmuskelgewebe bzw. im Trabekelwerk verankert werden, ohne einen Strom in das Gewebe einzuleiten. Der Strom wird von den Windungen 31 über die Länge des Kopfes 30 bereitgestellt und quert den Herzmuskel in seinem mittleren Bereich und nicht wie bei Schockelektroden im Verankerungsbereich.

Die Fig. 4 zeigt eine zweite Ausführungsform der Elektrode, bei der die Elektrode 30 aus einer oder mehreren metallischen, elektrisch leitenden Wendeln 31 und einem stumpfen distalen Ende 32, gebildet aus der oder den letzten Wendeln besteht. Hier steht vorteilhafterweise das nicht leitende Ende 32 der Elektrode über den Wendelbereich über, so dass bei einem losen Einlegen der Elektrode 30 in die rechte Herzkammer, diese Spitze durch ihre Vorschubposition oder ihr Gewicht in der Spitze der rechten Herzkammer aufliegt, aber eben, wie die Elektrode nach Fig. 3 keine direkten Einspeisung in diese Spitze der rechten Herzkammer bereitstellt. Die Wendel 31 ist bei der Elektrode nach Fig. 4 enger ausgestaltet, so dass nach dem Austritt aus dem Mantel der Elektrode die Wendel eine durchgängige leitende Fläche bereitstellt, ohne dass ein Durchblick auf den zentralen Kern besteht. Es ist somit eine gleichmässige Stromabgabe von einer zusammenhängenden Fläche möglich.

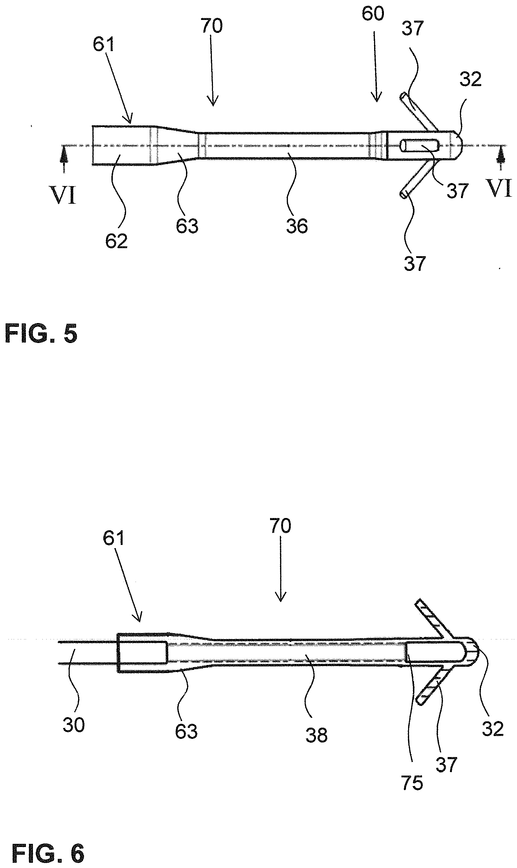

Die Fig. 5 zeigt eine skizzenhafte Darstellung einer Ankerspitze 60 einer Coil-Elektrode gemäss einem Ausführungsbeispiel der Erfindung zum Einsatz als Herzkammerelektrode nach Fig. 1 oder Fig. 2, und die Fig. 6 zeigt einen Querschnitt der Ankerspitze 60 entlang der Linie VI-VI in Fig. 5, welche auf eine Coil-Elektrodenspitze aufgesetzt ist.

Der Ankerbereich 60 umfasst eine Spitze mit einem stumpfen Ende 32 und von diesem stumpfen Ende 32 weg gerichteten vier Verankerungsspitzen 37. Anschliessend folgt ein Übergangsbereich 39, der eine Länge von 1 bis 3 Zentimeter haben kann. Dieser Übergangsbereich 39 geht dann über eine Verdickung 63 in einen flachzylindrischen Verbindungsbereich 62 über, die als distaler Verbinder mit der Elektrode fest verbunden sind. Der Anker 70 besteht vollständig aus elektrisch isolierendem Material, insbesondere einem Kunststoff.

Die Ankerspitzen 37 sind einstückig mit dem hohlzylindrischen Anker 70 verbunden, wie sich insbesondere aus dem Querschnitt der Fig. 6 ergibt, wobei sie hier um 45 Grad gegenüber der Längsachse des Ankers zurück gebogen sind, also in Richtung des leitenden Bereichs der Coilelektrode. Der Verbinder 61 ist auf einen in den hohlzylindrischen Anker 70 reichenden metallischen Frontabschnitt der Elektrode 30 aufgesetzt. Der Anker ist passiv auf der Elektrode fixiert. Die Endkappe 75 im vorderen Endbereich des Ankers 70, insbesondere im Bereich zwischen den Verankerungsspitzen 37 stabilisiert diese und schliesst das distale Ende der Elektrode ab, um zu vermeiden, dass ein eingesetzter Mandrin durch den Anker 70 hindurchstösst. Die Endkappe 75 hat einen Formschluss auf der Innenseite des Ankers 70, um die vorteilhafterweise durch Klebstoff realisierte Verbindung von Elektrodendraht, Endkappe 75, distaler Fixierung 61 und dem Anker 70 zu verstärken, so dass eine hohe Betriebsfestigkeit unter Zugbelastung besteht. Die Endkappe 75 selber ist hohlzylindrisch und nimmt die durchgeführte metallische Spitze der Elektrode in ihrem Innern auf.

**BEZUGSZEICHENLISTE**

| | | | |
|---|---|---|---|
| 10 | Herz | 55 | Stromflusspfeil |
| 20 | Elektrodenanordnung | 58 | einadrige Leitung |
| 30 | Coil-Herzkammer-Elektrode | 59 | Isolationsmantel |
| 31 | elektrisch leitende Wendel | 60 | Ankerbereich |
| 32 | stumpfes Ende | 61 | distaler Verbinder |
| 33 | Elektrodenspitzenkern | 62 | flachzylindrischer Verbinder |
| 35 | Austritt | 63 | Verdickung |
| 36 | Elektrodenspitze | 70 | Anker |
| 37 | Verankerungsspitzen | 75 | Endkappe |
| 38 | Kern | 140 | Flächenelektrode |
| 39 | Übergangsbereich | 141 | Kante der Flächenelektrode |
| 40 | Coil-Coronarsinus-Elektrode | 152 | einadrige Zuleitung |
| 50 | Steuerschaltung | 155 | Flächenpfeile |
| 51 | einadrige Zuleitung | | |
| 52 | einadrige Zuleitung | | |

## Patentansprüche

1. Implantierbare Gleichstromelektrodenanordnung (20, 120) mit zwei implantierbaren Elektroden (30; 140) und einer Steuerschaltung (50), wobei die erste und die zweite Elektrode (30; 140) mit der Steuerschaltung (50) elektrisch verbunden sind, wobei die Steuerschaltung (50) ausgestaltet ist, um zwischen den beiden Elektroden (30; 140) eine Potentialdifferenz aufzubauen, sodass zwischen den beiden Elektroden (30; 140) ein Gleichstrom (55; 155) fliessen kann, wobei die eine Elektrode (30) eine Coil-Elektrode ist, **dadurch gekennzeichnet, dass** die Gegen-Elektrode (140) eine Flächenelektrode zum Aufbringen auf der Aussenseite der linken Herzkammer ist.

2. Implantierbare Gleichstromelektrodenanordnung (20) nach Anspruch 1, **dadurch gekennzeichnet, dass** die elektrische Verbindung zwischen den ersten und zweiten Elektroden (30; 140) und der Steuerschaltung (50) als isolierte, einadrige Leitungen (51, 52, 152) vorgesehen sind.

3. Implantierbare Gleichstromelektrodenanordnung (20) nach einem der Ansprüche 1 oder 2, **dadurch gekennzeichnet, dass** die erste Coil-Elektrode (30) ausschliesslich aus einer oder mehreren Wendeln besteht, die um einen aus einem elektrisch isolierenden Material gefertigten Kern (33) der Elektrode gewunden sind, der einstückig mit dem isolierenden Mantel (59) der Zuleitung verbunden ist.

4. Implantierbare Gleichstromelektrodenanordnung (20) nach Anspruch 3, **dadurch gekennzeichnet, dass** das distale Ende (32) der ersten Coil-Elektrode (30) stumpf ist und über das Ende der Wendel(n) hinausragt.

5. Implantierbare Gleichstromelektrodenanordnung (20) nach Anspruch 3, **dadurch gekennzeichnet, dass** das distale Ende (32) der ersten Coil-Elektrode (30) aus mehreren voneinander abragenden freien Enden besteht, die über das Ende der Wendel(n) hinausragen.

6. Implantierbare Gleichstromelektrodenanordnung (20) nach Anspruch 5, **dadurch gekennzeichnet, dass** die freien Enden spitz zulaufen und/oder hakenförmig ausgestaltet sind.

7. Implantierbare Gleichstromelektrodenanordnung (20) nach einem der Ansprüche 1 bis 6, **dadurch gekennzeichnet, dass** die Coil-Elektrode (30) eine durch den Abstand zwischen Trikuspidalklappe und dem Trikuspidalklappe und Pulmonalklappe gegenüberliegenden Ende der rechten Herzkammer vorbestimmte maximale Länge zwischen 4 und 12 Zentimeter aufweist.

8. Implantierbare Gleichstromelektrodenanordnung (20) nach Anspruch 7, **dadurch gekennzeichnet, dass** die Coil-Elektrode (30) eine Länge zwischen 6 und 10 Zentimeter hat.

## Claims

1. An implantable direct-current electrode assembly (20, 120) comprising two implantable electrodes (30; 140) and a control circuit (50), wherein the first and second electrodes (30; 140) are electrically connected to the control circuit (50), wherein the control circuit (50) is adapted to establish a potential difference between the two electrodes (30; 140) such that a direct current (55; 155) can flow between the two electrodes (30; 140), wherein the one electrode (30) is a coil electrode, **characterized in that** the counter electrode (140) is a surface electrode for application to the outside of the left ventricle.

2. The implantable direct-current electrode assembly (20) according to claim 1, **characterized in that** the electrical connection between the first and second electrodes (30; 140) and the control circuit (50) are provided as insulated single-conductor leads (51, 52, 152).

3. The implantable direct-current electrode assembly (20) according to one of claims 1 or 2, **characterized in that** the first coil electrode (30) consists exclusively of one or more windings wound around an electrode core (33) which is connected integrally to the insulating sheath (59) of the lead.

4. The implantable direct-current electrode assembly (20) according to claim 3, **characterized in that** the distal end (32) of the first coil electrode (30) is blunt and projects beyond the end of the winding(s).

5. The implantable direct-current electrode assembly (20) according to claim 3, **characterized in that** the distal end (32) of the first coil electrode (30) is composed of a plurality of spaced apart free ends and projecting beyond the end of the winding(s).

6. The implantable direct-current electrode arrangement (20) according to Claim 5, **characterized in that** the free ends are tapered to a point and/or hook-shaped.

7. The implantable direct-current electrode assembly (20) according to any of claims 1 to 6, **characterized in that** the coil electrode (30) has a maximum length of between 4 and 12 centimetres predetermined by the distance between the tricuspid valve and the tricuspid valve and pulmonary valve opposite the end of the right ventricle.

8. The implantable direct-current electrode assembly (20) according to claim 7, **characterized in that** the coil electrode (30) has a length between 6 and 10 centimeters.

## Revendications

1. Ensemble d'électrodes implantable à courant continu (20, 120) comprenant deux électrodes implantables (30; 140) et un circuit de commande (50), les premières et secondes électrodes (30; 140) étant connectées électriquement au circuit de commande (50), dans lequel le circuit de commande (50) est adapté pour établir entre les deux électrodes (30; 140) une différence de potentiel de sorte qu'un courant continu (55; 155) puisse circuler entre les deux électrodes (30; 140), où l'une des électrodes (30) est une électrode de bobine, **caractérisé en ce que** la contre-électrode (140) est une électrode plane pour une application à l'extérieur du ventricule gauche.

2. Ensemble d'électrodes implantable à courant continu (20) selon la revendication 1, **caractérisé en ce que** la connexion électrique entre les premières et deuxièmes électrodes (30; 140) et le circuit de commande (50) est prévue sous la forme de conducteurs monoconducteurs isolés (51, 52, 152).

3. Ensemble d'électrodes implantable à courant continu (20) selon l'une des revendications 1 ou 2, **caractérisé en ce que** la première électrode de bobine (30) est constituée exclusivement d'une ou plusieurs bobines enroulées autour d'un noyau (33) de l'électrode en un matériau électriquement isolant relié intégralement à la gaine isolante (59) de l'alimentation.

4. Ensemble d'électrodes implantable à courant continu (20) selon la revendication 3, **caractérisé en ce que** l'extrémité distale (32) de la première électrode de bobine (30) est émoussée et dépasse de l'extrémité de la ou des bobines.

5. Ensemble d'électrodes implantable à courant continu (20) selon la revendication 3, **caractérisé en ce que** l'extrémité distale (32) de la première électrode de bobine (30) consiste en une pluralité d'extrémités libres espacées l'une de l'autre dépassant l'extrémité de la ou des bobines.

6. Ensemble d'électrodes implantable à courant continu (20) selon la revendication 5, **caractérisé en ce que** les extrémités libres se terminent en pointes et/ou en forme de crochet.

7. Ensemble d'électrodes implantable à courant continu (20) selon l'une quelconque des revendications 1 à 6, **caractérisé en ce que** l'électrode à bobine (30) a une longueur maximale comprise entre 4 et 12 centimètres prédéterminée par la distance entre la valve tricuspide et la valve tricuspide et la valve pulmonaire en face de l'extrémité du ventricule droit.

8. Ensemble d'électrodes implantable à courant continu (20) selon la revendication 7, **caractérisé en ce que** l'électrode de bobine (30) a une longueur comprise entre 6 et 10 centimètres.
